# EUROPEAN PATENT SPECIFICATION

(11) **EP 3 318 206 B1**
(45) Date of publication and mention of the grant of the patent: **02.11.2022**
(21) Application number: 16818264.0
(22) Date of filing: 30.06.2016
(51) Int. Cl.: A61B 17/32, A61B 17/3205, A61B 17/34, A61B 18/14, A61B 17/00, A61B 18/00

(54) **DEVICE FOR TISSUE REMOVAL**
VORRICHTUNG ZUR GEWEBEENTFERNUNG
DISPOSITIF DE RETRAIT DE TISSU

(30) Priority: 30.06.2015 KR 20150093457
(43) Date of publication of application: 09.05.2018
(73) Proprietor: Soonchunhyang University Industry Academy Cooperation Foundation, Asan-si, Chungcheongnam-do 31358 (KR)
(72) Inventor: RYU, Chang Beom, Seongnam-si Gyeonggi-do 13522 (KR)
(74) Representative: Plougmann Vingtoft a/s
(86) International application number: PCT/KR2016/007065
(87) International publication number: WO 2017/003231

(56) References cited:
- WO-A1-2014/196746
- JP-A- 2009 240 380
- JP-A- 2010 252 967
- JP-B2- 4 692 141
- KR-A- 20080 013 945
- KR-B1- 100 596 554
- KR-B1- 101 056 698
- KR-B1- 101 328 473
- US-A1- 2007 038 213

## Description

### [Technical field]

The present invention relates to a tissue removal device.

### [Background]

If a cancer in an esophagus, a stomach, and a large intestine which are human digestive organs is detected in early stages due to a quantum leap in an endoscopy device and therapeutic endoscopy, it can be completely cured by an endoscopic treatment. This provides not only recovering the quality of life but also a complete cure to a patient.

Among these, a procedure which is applied to an early gastric cancer limited to a mucousa or limited submucosa, an early colorectal cancer and an early esophageal cancer and is used as a standard treatment in Korea and Japan is endoscopic submucosal dissection (ESD).

ESD procedure is performed after detecting a lesion such as an early cancer by having endoscope, taking a biopsy and receiving definite diagnosis, and during the procedure, the most important tool is an incision knife. This incision knife is used to mark by energizing a nearby lesion, to inject saline solution and epinephrine mixture to a submucousa, to remove a marginal zone of early cancer and the submucousa, and to remove the area of an early cancer.

However, the existing incision knives are mostly made in Japan and have merits and drawbacks. Particularly, during the procedure, the additional injection of saline solution and epinephrine mixture to the submucousa is repeatedly needed while a mucousa or a submucousa is being cut by the incision knife. At this time, the mucous membrane and the submucous a are cut by the steps of removing the incision knife, inserting an injection catheter through a working channel, injecting saline solution and epinephrine mixture to the submucousa through the infusion cannula, removing the infusion cannula, and inserting the incision knife again through the biopsy channel. Also, when the mucousa or the submucousa is cut, if the contact angle of the incision knife and the marginal zone to be cut is not right or the incision knife is hard to contact with the marginal zone, another type of electrosurgical knives are replaced and inserted multiple times. It may cause a longer procedure time and an increase of a patient's pain and a doctor's fatigue.

Accordingly, the necessity of an incision knife that can cut from various angles without removing an infusion cannula and with saline solution and epinephrine mixture being simultaneously injected is on the rise.

WO 2014/196746 A1 describes an endoscopic treatment device comprising a tube, a dome knife wire being connected to a handle with one end and, with the other end, to a dome knife having a dome shaped end that protrudes from the tube, and needle knife wire connected with one end to a needle knife.

JP 2009 240380 A describes a treatment device comprising a first electrode serving as an incising stripping knife and a second electrode serving as a needle-shaped knife. The second electrode is inserted into the first electrode, wherein both electrodes can be protruded from a tip of a tube independently from each other.

### [CONTENTS OF THE INVENTION]

### [Technical Object]

The present invention has been made in an effort to provide a tissue removal device having advantages of removing an abnormal tissue at various positions without replacing an incision knife of the tissue removal device.

### [Means for achieving the object]

A tissue removal device according to an exemplary embodiment of the present invention may include a tube with a predetermined length, an operation unit connected with one side of the tube, a first wire which is located inside of the tube and has one side connected with the operation unit, a first incision knife which is connected with the other side of the first wire, wherein at least a part of the first incision knife can be drawn out to an outside of the tube, a ball which is positioned outside of the tube and is connected to a tip of the first incision knife, a second wire which is disposed inside of the tube and has one side connected with the operation unit, and a second incision knife which is connected with the other side of the second wire, penetrates through the first incision knife, and can be protruded from the ball, wherein the first incision knife is movable with the first wire by operation of the operation unit. According to the invention, the operation unit includes a body connected with the tube and having an inside being empty, a rail connected with the body, and a slide movably connected with the rail and connected with the first wire, wherein one side of the second wire is drawn out of the body. A first inlet connected with the inside of the body is formed on a surface of the body, and the inside of the body may be connected with the inside of the tube. The tissue removal device further includes an injection body connected with the one side of the second wire drawn out of the operation unit and having a second inlet, wherein pathways are formed inside of the second wire and inside of the second incision knife and are connected with each other, and the pathway of the second wire is connected with the second inlet.

The tissue removal device may further include a first stopper connected with the first incision knife and having a diameter larger than that of the first incision knife, wherein the first incision knife penetrates through a blocking ring disposed in the tube, and the first stopper can be blocked by the blocking ring such that a length by which the first incision knife is drawn out is limited.

The tissue removal device may further include a guide ring spaced from the first incision knife, coupled with the first wire and having an inside through which the second wire penetrates, and a second stopper positioned between the first incision knife and the guide ring and coupled to the second incision knife, wherein a moving distance of the second incision knife is limited by the second stopper moving between the guide ring and the first incision knife.

### [Effect of the Invention]

According to an exemplary embodiment of the present invention, using the first incision knife between the tube and the ball, the abnormal tissue which is parallel or oblique to the first incision knife can be removed. When the abnormal tissue cannot contact with the first incision knife to difficult contact angle between first incision knife and target tissue, the abnormal tissue can be removed by protruding the second wire outside of the ball, and thereby causing an operation easier without replacing wires.

### [BRIEF DESCRIPTION OF THE DRAWINGS]

FIG. 1 is a perspective view of a tissue removal device according to an exemplary embodiment of the present invention.
FIG. 2 is an enlarged perspective view of an operation unit of FIG. 1.
FIG. 3 is an enlarged view of part A in FIG. 1.
FIG. 4 is a cross-sectional view taken along the line IV-IV in FIG. 3.
FIG. 5 is a cross-sectional view showing the condition that a first incision knife in FIG. 4 is drawn out outside of a tube.
FIG. 6 is a cross-sectional view showing the condition that a second incision knife is drawn out from a first incision knife in FIG. 5.
FIG. 7 is a perspective view showing the condition that a second incision knife is drawn out from the condition of FIG. 4.

### [Modes for executing the invention]

The present invention will be described more fully hereinafter with reference to the accompanying drawings, in which exemplary embodiments of the invention are shown. As those skilled in the art would realize, the described embodiments may be modified in various different ways, all without departing from the scope of the present invention defined by the claims. Like reference numerals designate like elements throughout the specification.

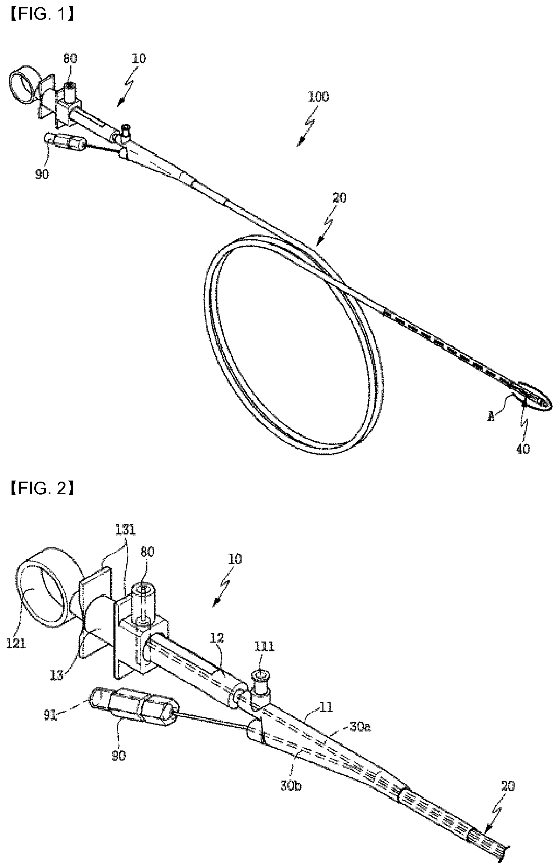

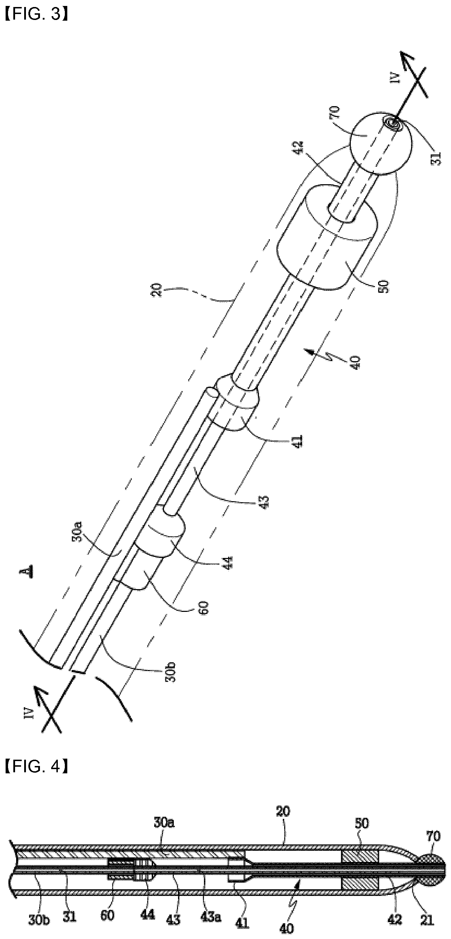

Then, a tissue removal device according to an exemplary embodiment of the present invention will be described referring to FIG. 1 to FIG. 4. FIG. 1 is a perspective view of a tissue removal device according to an exemplary embodiment of the present invention, FIG. 2 is an enlarged perspective view of an operation unit of FIG. 1, FIG. 3 is an enlarged view of part A in FIG. 1, and FIG. 4 is a cross-sectional view taken along the line IV-IV.

Referring to FIG. 1 to FIG. 4, a tissue removal device 100 according to the present exemplary embodiment includes an operation unit 10, a tube 20, a first wire 30a, a second wire 30b, an incision unit 40, a ball 70, a blocking ring 50, a guide ring 60, a current terminal 80 and an injection body 90.

An operation unit 10 is hand holdable and includes a body 11, a rail 12 and a slide 13.

The body 11 has a predetermined length and an inside of the body 11 is empty. On a surface of the body 11, a first inlet 111 connected with the inside is formed. Saline solution and epinephrine mixture, etc. can be injected to the inside of the body 11 through the first inlet 111.

The rail 12 has a predetermined length and is connected with the body 11. A ring 121 into which a user can put a finger is formed at one side of the rail 12.

The slide 13 encloses a part of the rail 12, and can move along a length direction of the rail 12. A catching plate 131 that a user can put a finger on is formed on the slide 13. The current terminal 80 to which a cable (not shown) for supplying high-frequency current can be connected is coupled with the slide 13.

The tube 20 has a predetermined length and has one side connected with the body 11. An inside of the tube 20 is connected with the inside of the body 11. Saline solution and epinephrine mixture, etc. that is injected to the inside of the body 11 can flow into the tube 20.

The other side of the tube 20 can be positioned in an esophagus, a stomach or a large intestine, etc. through an endoscope (not shown). An open hole 21 is formed at the other side of the tube 20, and a diameter of the open hole 21 is smaller than that of an internal circumference of the tube 20. Saline solution and epinephrine mixture, etc. which is flowed into the tube 20 can be discharged to an outside of the tube 20 through the open hole 21.

The first wire 30a has a predetermined length and is disposed inside of the tube 20. A side of the first wire 30a penetrates the body 11 and is connected with the current terminal 80. The first wire 30a can move with the motion of the slide 13. The first wire 30a may be made of electric conductive material.

The second wire 30b has a predetermined length and is disposed inside of the tube 20. The second wire 30b contacts with the first wire 30a and may be made of the same material as the first wire 30a. Since the first wire 30a and the second wire 30b are electrically connected with each other, current supplied to the first wire 30a can be supplied to the second wire 30b.

A pathway 31 is formed inside of the second wire 30b along the length direction thereof. The injection body 90 that can push and pull the second wire 30b is connected to one side of the second wire 30b. A second inlet 91 connected with the pathway 31 is formed at the injection body 90, and saline solution or epinephrine mixture etc. injected through the second inlet 91 can flow into the pathway 31.

A sealing member (not shown) is disposed in the body 11 which the first wire 30a and the second wire 30b pass through. The sealing member prevents saline solution injected through the first inlet 111 from leaking between the wires 30a and 30b and the body 11.

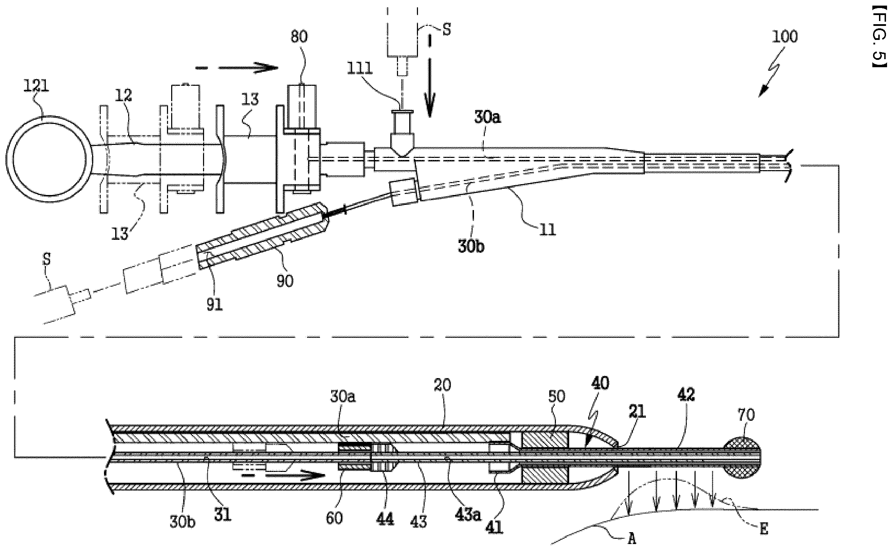

The incision unit 40 is to remove an abnormal tissue E in an esophagus, a stomach, or a large intestine, etc. (referring to FIG. 5 to FIG. 7), and includes a first stopper 41, a first incision knife 42, a second incision knife 43 and a second stopper 44. The incision unit 40 can be made of electric conductive material.

The first stopper 41 is disposed inside of the tube 20 and is connected with the other side of the first wire 30a. An inside of the first stopper 41 is penetrated along the length direction thereof. A diameter of an internal circumference of the first stopper 41 becomes smaller from one side toward the other side of the first stopper 41. The first stopper 41 can be removed.

The first incision knife 42 has a predetermined length and is disposed inside of the tube 20. One side of the first incision knife 42 is connected with the other side of the first stopper 41. At least a part of the other side of the first incision knife 42 is exposed outside of the tube 20 through the open hole 21.

A diameter of an external circumference of the first incision knife 42 is smaller than that of an external circumference of the first stopper 41. An inside of the first incision knife 42 is empty along the length direction, and is connected with the inside of the first stopper 41. In case that the first stopper 41 is omitted, the first incision knife 42 can be connected with the first wire 30. The first incision knife 42 can be drawn out from the inside of the tube 20 to the outside through the open hole 21 or can be inserted back into the inside of the tube 20 in a condition that the first incision knife 42 is drawn out to the outside according to the movement of the first wire 30a.

The ball 70 is positioned outside of the tube 20 and is coupled with the external circumference of the first incision knife 42. A tip of the first incision knife 42 is not protruded from a circumference of the ball 70. The diameter of the ball 70 is larger than that of the internal circumference of the tube 20. The ball 70 can be detached from or attached to the tube 20 according the movement of the first incision knife 42.

The distance that the ball 70 is detached from the other side of the tube 20 is about 3mm to 4mm. Accordingly, a length by which the first incision knife 42 can be drawn out is about 3mm to 4mm. The length by which the first incision knife 42 can be drawn out may be variously changed according to a design of the tissue removal device 100. The abnormal tissue E can be removed by contacting the first incision knife 42 between the ball 70 and the tube 20an with the abnormal tissue E.

The second incision knife 43 is disposed inside of the first incision knife 42 and is connected with the other side of the second wire 30b. A diameter of an external circumference of the second incision knife 43 is smaller than that of an internal circumference of the first incision knife 42. A pathway 43a connected with the pathway 31 is formed inside of the second incision knife 43. Saline solution or epinephrine mixture flowing into the pathway 31 can be discharged outside of the second incision knife 43 through the pathway 43a.

According to the movement of the second wire 30b, the second incision knife 43 can be drawn out of the first incision knife 42 and protruded from the ball 70, or inserted into the inside of the first incision knife 42 in a condition that the second incision knife 43 is protruded. A length by which the second incision knife 43 can be protruded from the ball 70 is about 1mm to 2mm. The length by which the second incision knife 43 can be protruded from the ball 70 may be variously changed according to a design of the tissue removal device 100.

The blocking ring 50 is fixed to the inside of the tube 20 and the first incision knife 42 penetrates through the blocking ring 50. A gap is formed between an internal circumference of the blocking ring 50 and the external circumference of the first incision knife 42. The movement of the first stopper 41 can be limited by the blocking ring 50. The length by which the first incision knife 42 can be drawn out is limited by the first stopper 41 and the blocking ring 50. However, the blocking ring 50 may be removed. In this case, the movement of the first stopper 41 may be limited by the surrounding area of the open hole 21 of the tube 20.

The guide ring 60 is spaced from the first stopper 41 and is connected with the first wire 30a. The guide ring 60 guides the other side of the second wire 30b to move straight in the other side of the tube 20. Due to the straight movement of the second wire 30b, the second incision knife 43 can also move straight.

The second stopper 44 is positioned between the first incision knife 42 and the guide ring 60 and is connected with the second incision knife 43. According to the movement of the second wire 30b, the second stopper 44 contacts with the guide ring 60 or the first stopper 41. Because the second stopper 44 moves between the guide ring 60 and the first stopper 41, the range of movement of the second wire 30b and the second incision knife 43 is set between the guide ring 60 and the first stopper 41.

On condition that the second stopper 44 contacts with the guide ring 60 or the first stopper 41, if the first wire 30a moves, the second wire 30b can move with the first wire 30a. Therefore, the second incision knife 43 can move with the first incision knife 42.

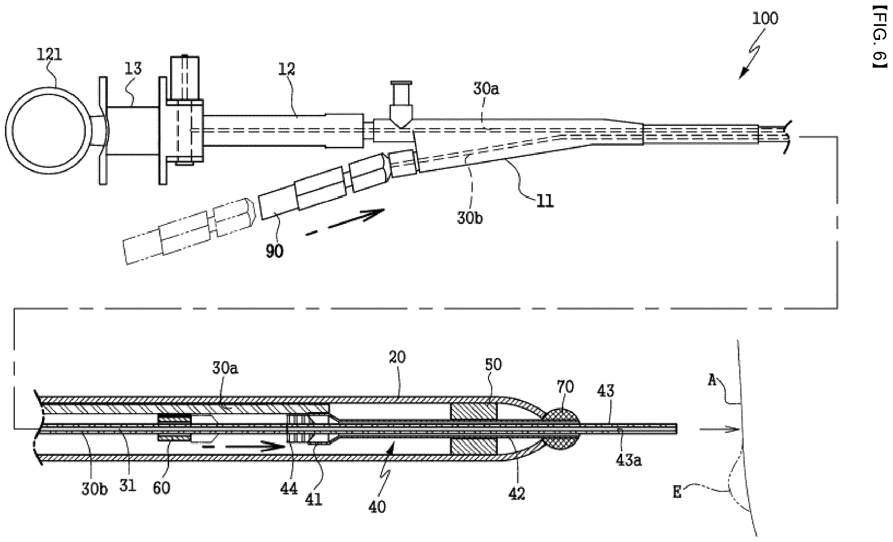

Next, referring to FIG. 5 and FIG. 6, the operation of the tissue removal device will be described.

FIG. 5 is a cross-sectional view showing the condition that a first incision knife in FIG. 4 is drawn out of the outside of a tube, and FIG. 6 is a cross-sectional view showing the condition that a second incision knife is drawn out of a first incision knife in FIG. 5.

Firstly, referring to FIG. 5, when the other side of the tube 20 reaches the abnormal tissue E which is located in a parallel or slanted position to the external circumference of the first incision knife 42 on the condition that the incision unit 40 is positioned inside of the tube 20, a user pushes the slide 13 away from the ring 121.

As the first wire 30a moves toward the other side of the tube 20 by the slide 13, the first incision knife 42 is drawn out and the ball 70 is detached from the tube 20. If the first stopper 41a contacts with the blocking ring 50, the first incision knife 42 is not drawn out any more.

If the first incision knife 42 is drawn out, the abnormal tissue E is removed by using the external circumference of the first incision knife 42 between the ball 70 and the tube 20. The abnormal tissue E is removed by current supplied to the first wire 30a through the current terminal 80. Additionally, the tip of the first incision knife 42 does not stab in a normal tissue by the ball 70.

After removing the abnormal tissue E, when the slide 13 is pulled toward the ring 121, the first wire 30a is pulled and the first incision knife 42 is inserted into the inside of the tube 20. If the ball 70 contacts with the tube 20, the first incision knife 42 is not further inserted into the inside of the tube 20.

In the meantime, when the first wire 30a moves toward the other side of the tube 20 as the guide ring 60 contacts the second stopper 44, the second wire 30a can move along the first wire 30a. Accordingly, the second incision knife 43 can move along the first incision knife 42.

Next, the operation of the tissue removal device will be described referring to FIG. 6. If the abnormal tissue E faces the tip of the first incision knife 42 instead of the external circumference of the first incision knife 42 that is drawn out, the second wire 30b is moved by pushing the injection body 90 toward the body 11.

Due to the movement of the second wire 30b, the second incision knife 43 is drawn out of the first incision knife 42 and is protruded from the ball 70. If the second stopper 44 is stuck in the first stopper 41, the second incision knife 43 is not protruded any more. Using the protruded tip of the second incision knife 43, the abnormal tissue E is removed from a mucous membrane A (please refer to FIG. 5 to FIG. 7). The abnormal tissue E is removed by current supplied to the second wire 30b through the first terminal 80.

After removing the tissue, if the injection body 90 is pulled away from the body 11, the second wire 30b is pulled and the second incision knife 43 is positioned inside of the first incision knife 42. If the second stopper 44 contacts with the guide ring 60, the second incision knife 43 is not inserted any more. In other words, the tip of the second incision knife 43 is positioned inside of the first incision knife 42.

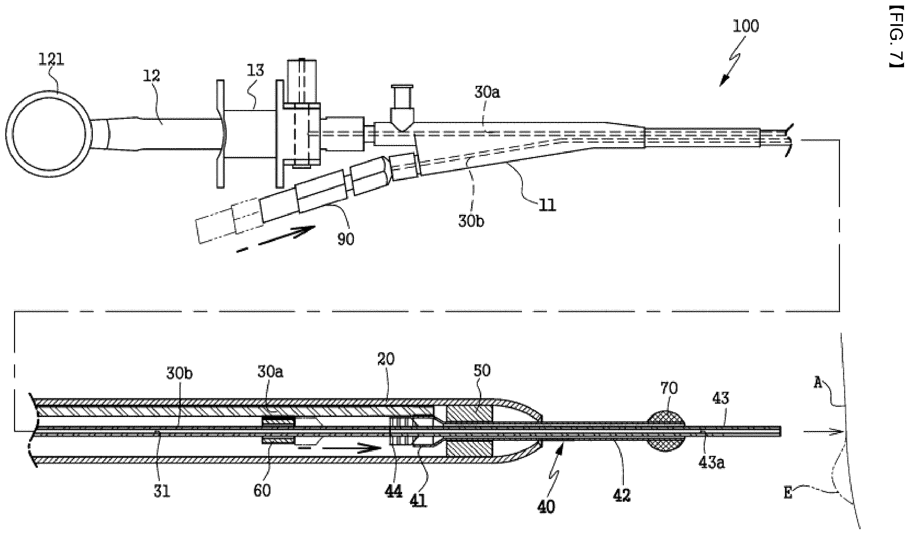

In the meantime, as shown in FIG. 7, on the condition that the first incision knife 42 is positioned in the tube 20, the second incision knife 43 is drawn out to the outside of the first incision knife 42 and can remove the abnormal tissue E.

Referring to FIG. 5 again, if saline solution and epinephrine mixture is injected to the second inlet 91 using a syringe S and so on during the incision of the abnormal tissue E, saline solution and epinephrine mixture flows through pathways 31 and 43a and can be discharged toward the abnormal tissue E from the second incision knife 43. In case that the second incision knife 43 is drawn out of the first incision knife 42 and the tip of the second incision knife 43 is inserted in the abnormal tissue E, saline solution and epinephrine mixture can be injected into the abnormal tissue E. The abnormal tissue E may swell by saline solution and epinephrine mixture.

Meanwhile, saline solution and epinephrine mixture can be injected into the tube 20 through the first inlet 111. Saline solution flows through the tube 20 and can be discharged toward the abnormal tissue E at the open hole 21. In case that the first inlet 111 is connected with the inside of the incision unit 40, saline solution can be sprayed from the incision unit 40.

Another exemplary embodiment of the present disclosure not covered by the present invention mostly includes the constituent elements of the exemplary embodiment described referring to FIG. 1 to FIG. 7. However, the pathway 43a in the second incision knife 43 is removed. The pathway 31 of the second wire 30a is connected with the inside of the first incision knife 42. Therefore, saline solution and epinephrine mixture injected through the second inlet 91 can be discharged to the abnormal tissue E through the first incision knife 42. The other constituent elements of the exemplary embodiment in FIG. 1 to FIG. 7 may be applied to another exemplary embodiment as they are.

While this invention has been described in connection with what is presently considered to be practical exemplary embodiments, it is to be understood that the invention is not limited to the disclosed embodiments, but, on the contrary, is intended to cover various modifications included within the scope of the appended claims.

## Claims

1. A tissue removal device (100) comprising:
a tube (20) with a predetermined length,
an operation unit (10) connected with one side of the tube (20),
a first wire (30a) which is located inside of the tube (20) and has one side connected with the operation unit (10),
a first incision knife (42) which is connected with the other side of the first wire (30a), wherein at least a part of the first incision knife (42) can be drawn out to an outside of the tube (20),
a ball (70) which is positioned outside of the tube (20) and is connected to a tip of the first incision knife (42),
a second wire (30b) which is disposed inside of the tube (20) and has one side connected with the operation unit (10), and
a second incision knife (43) which is connected with the other side of the second wire (30b), penetrates through the first incision knife (42), and can be protruded from the ball (70), wherein the first incision knife (42) is movable with the first wire (30a) by operation of the operation unit (10), wherein the operation unit (10) comprises a body (11) connected with the tube (20) and having an inside being empty, a rail (12) connected with the body (11), and a slide (13) movably connected with the rail (12) and connected with the first wire (30a), wherein one side of the second wire (30b) is drawn out of the body (11), wherein a first inlet (111) connected with the inside of the body (11) is formed on a surface of the body (11), and the inside of the body (11) is connected with the inside of the tube (20),
an injection body (90) connected with the one side of the second wire (30b) drawn out of the operation unit (10) and having a second inlet (91),
wherein pathways (31, 43a) are formed inside of the second wire (30b) and inside of the second incision knife (43) and are connected with each other, and the pathway (31) of the second wire (30b) is connected with the second inlet (91).

2. The tissue removal (100) device of claim 1, further comprising a first stopper (41) connected with the first incision knife (42) and having a diameter larger than that of the first incision knife (42),
wherein the first incision knife (42) penetrates through a blocking ring (50) disposed in the tube (20), and the first stopper (41) can be blocked by the blocking ring (50) such that a length by which the first incision knife (42) is drawn out is limited.

3. The tissue removal device (100) of claim 1, further comprising a guide ring (60) spaced from the first incision knife (42), coupled with the first wire (30a) and having an inside through which the second wire (30b) penetrates, and a second stopper (44) positioned between the first incision knife (42) and the guide ring (60) and coupled to the second incision knife (43),
wherein a moving distance of the second incision knife (43) is limited by the second stopper (44) moving between the guide ring and the first incision knife (42).

## Patentansprüche

1. Gewebeentfernungsvorrichtung (100), umfassend:
einen Schlauch (20) mit einer vorbestimmten Länge,
eine Betätigungseinheit (10), die mit einer Seite des Schlauchs (20) verbunden ist,
einen ersten Draht (30a), der sich innerhalb des Schlauchs (20) befindet und eine Seite aufweist, die mit der Betätigungseinheit (10) verbunden ist,
ein erstes Schneidmesser (42), das mit der anderen Seite des ersten Drahts (30a) verbunden ist, wobei mindestens ein Teil des ersten Schneidmessers (42) nach außen aus dem Schlauch (20) herausgezogen werden kann,
eine Kugel (70), die außerhalb des Schlauchs (20) positioniert ist und mit einer Spitze des ersten Schneidmessers (42) verbunden ist,
einen zweiten Draht (30b), der innerhalb des Schlauchs (20) angeordnet ist und eine Seite aufweist, die mit der Betätigungseinheit (10) verbunden ist, und
ein zweites Schneidmesser (43), das mit der anderen Seite des zweiten Drahtes (30b) verbunden ist, das erste Schneidmesser (42) durchdringt und aus der Kugel (70) herausgestreckt werden kann, wobei das erste Schneidmesser (42) mit dem ersten Draht (30a) durch Betätigung der Betätigungseinheit (10) bewegbar ist, wobei die Betätigungseinheit (10) einen Körper (11), der mit dem Schlauch (20) verbunden ist und ein leeres Inneres aufweist, eine Schiene (12), die mit dem Körper (11) verbunden ist, und einen Schlitten (13), der bewegbar mit der Schiene (12) verbunden ist und mit dem ersten Draht (30a) verbunden ist, umfasst, wobei eine Seite des zweiten Drahts (30b) aus dem Körper (11) herausgezogen ist, wobei ein erster Einlass (111), der mit dem Inneren des Körpers (11) verbunden ist, an einer Oberfläche des Körpers (11) ausgebildet ist und das Innere des Körpers (11) mit dem Inneren des Schlauchs (20) verbunden ist,
einen Einspritzkörper (90), der mit der einen Seite des zweiten Drahts (30b) verbunden ist, die aus der Betätigungseinheit (10) herausgezogen ist, und der einen zweiten Einlass (91) aufweist,
wobei Kanäle (31, 43a) innerhalb des zweiten Drahtes (30b) und innerhalb des zweiten Schneidmessers (43) ausgebildet und miteinander verbunden sind und der Kanal (31) des zweiten Drahtes (30b) mit dem zweiten Einlass (91) verbunden ist.

2. Gewebeentfernungsvorrichtung (100) nach Anspruch 1, ferner umfassend einen ersten Stopper (41), der mit dem ersten Schneidmesser (42) verbunden ist und einen Durchmesser aufweist, der größer als der des ersten Schneidmessers (42) ist,
wobei das erste Schneidmesser (42) einen in dem Schlauch (20) angeordneten Sperrring (50) durchdringt und der erste Stopper (41) durch den Sperrring (50) so blockiert werden kann, dass eine Länge, um die das erste Schneidmesser (42) herausgezogen wird, begrenzt ist.

3. Gewebeentfernungsvorrichtung (100) nach Anspruch 1, ferner umfassend einen Führungsring (60), der von dem ersten Schneidmesser (42) beabstandet ist, mit dem ersten Draht (30a) gekoppelt ist und ein Inneres aufweist, das der zweite Draht (30b) durchdringt, und einen zweiten Stopper (44), der zwischen dem ersten Schneidmesser (42) und dem Führungsring (60) positioniert ist und mit dem zweiten Schneidmesser (43) gekoppelt ist,
wobei eine Bewegungsstrecke des zweiten Schneidmessers (43) durch den zweiten Stopper (44), der sich zwischen dem Führungsring und dem ersten Schneidmesser (42) bewegt, begrenzt ist.

## Revendications

1. Dispositif de retrait de tissu (100), comprenant :
un tube (20) d'une longueur prédéterminée,
une unité d'actionnement (10) reliée à un côté du tube (20),
un premier fil (30a) qui est situé à l'intérieur du tube (20) et dont un côté est relié à l'unité d'actionnement (10),
un premier couteau à inciser (42) qui est relié à l'autre côté du premier fil (30a), dans lequel au moins une partie du premier couteau à inciser (42) peut être tirée jusqu'à l'extérieur du tube (20),
une bille (70) qui est positionnée à l'extérieur du tube (20) et est reliée à une pointe du premier couteau à inciser (42),
un second fil (30b) qui est disposé à l'intérieur du tube (20) et dont un côté est relié à l'unité d'actionnement (10), et
un second couteau à inciser (43) qui est relié à l'autre côté du second fil (30b), pénètre à travers le premier couteau à inciser (42) et peut dépasser de la bille (70), dans lequel le premier couteau à inciser (42) est mobile avec le premier fil (30a) par actionnement de l'unité d'actionnement (10), dans lequel l'unité d'actionnement (10) comprend un corps (11) relié au tube (20) et ayant un intérieur vide, un rail (12) relié au corps (11), et un coulisseau (13) relié de manière mobile au rail (12) et relié au premier fil (30a), dans lequel un côté du second fil (30b) est tiré hors du corps (11), dans lequel une première entrée (111) reliée à l'intérieur du corps (11) est formée sur une surface du corps (11), et l'intérieur du corps (11) est relié à l'intérieur du tube (20),
un corps d'injection (90) relié au premier côté du second fil (30b) tiré hors de l'unité d'actionnement (10) et ayant une seconde entrée (91),
dans lequel des trajets (31, 43a) sont formés à l'intérieur du second fil (30b) et à l'intérieur du second couteau à inciser (43) et sont reliés l'un à l'autre, et le trajet (31) du second fil (30b) est relié à la seconde entrée (91).

2. Dispositif de retrait de tissu (100) selon la revendication 1, comprenant en outre une première butée (41) reliée au premier couteau à inciser (42) et ayant un diamètre supérieur à celui du premier couteau à inciser (42),
dans lequel le premier couteau à inciser (42) pénètre à travers un anneau de blocage (50) disposé dans le tube (20), et la première butée (41) peut être bloquée par l'anneau de blocage (50) de sorte qu'une longueur sur laquelle le premier couteau à inciser (42) est tiré soit limitée.

3. Dispositif de retrait de tissu (100) selon la revendication 1, comprenant en outre un anneau de guidage (60) espacé du premier couteau à inciser (42), couplé au premier fil (30a) et ayant un intérieur à travers lequel le second fil (30b) pénètre, et une seconde butée (44) positionnée entre le premier couteau à inciser (42) et la bague de guidage (60) et couplée au second couteau à inciser (43),
dans lequel une distance de déplacement du second couteau à inciser (43) est limitée par la seconde butée (44) se déplaçant entre l'anneau de guidage et le premier couteau à inciser (42).
